# FASCICULE DE BREVET EUROPEEN

(11) **EP 0 648 270 B1**
(45) Date de publication et mention de la délivrance du brevet: **28.12.2005**
(21) Numéro de dépôt: 93913130.6
(22) Date de dépôt: 15.06.1993
(51) Int. Cl.: C12N 15/30, C12N 1/21, C12P 21/02, C12N 15/62, G01N 33/566, A61K 39/002, C12N 5/10

(54) **CLONAGE DU GENE CODANT POUR LA PROTEINE GP28.5 DE T. GONDII; FRAGMENTS PEPTIDIQUES ISSUS DE LADITE PROTEINE ET LEURS APPLICATIONS**
KLONERING VON DEM GEN FÜR DAS T GONDII PROTEIN GP 28.5 PEPTIDFRAGMENTE AUS DIESEM PROTEIN UND IHRE VERWENDUNGEN.
CLONING OF GENE ENCODING THE GP28.5 PROTEIN OF TOXOPLASMA GONDII; PEPTIDE FRAGMENTS OF SAID PROTEIN AND THEIR APPLICATIONS

(30) Priorité: 15.06.1992 FR 9207206
(43) Date de publication de la demande: 19.04.1995
(73) Titulaire: INSTITUT PASTEUR DE LILLE, 59019 Lille Cédex (FR); INSTITUT NATIONAL DE LA SANTE ET DE LA RECHERCHE MEDICALE (INSERM), 75654 Paris Cédex 13 (FR)
(72) Inventeur: CESBRON, Marie-France, F-59700 Marcq-en-Baroeul (FR); MERCIER, Corinne, F-59177 Sains-du-Nord (FR); CAPRON, André, F-59133 Phalempin (FR); TARTAR, André, F-62490 Vitry-en-Artois (FR); MAES, Pierrette, F-59290 Wasquehal (FR)
(74) Mandataire: Orès, Bernard
(86) Numéro de dépôt international: PCT/FR1993/000575
(87) Numéro de publication internationale: WO 1993/025689

(56) Documents cités:
- US-A- 5 686 575
- MOLECULAR AND BIOCHEMICAL PARASITOLOGY vol. 34, 1989, pages 3 - 14 J. B. PRINCE ET AL 'Cloning of cDNAs encoding a 28 kilodalton antigen of Toxoplasma gondii' cité dans la demande
- PARASITOLOGY vol. 103, no. 3, 1991, LONDON pages 321 - 329 A. ACHBAROU ET AL 'Differential targeting of dense granule proteins in the parasitophorous vacuole of Toxoplasma gondii'
- EXPERIMENTAL PARASITOLOGY vol. 71, 1990, pages 114 - 124 H. CHARIF ET AL 'Toxoplasma gondii : Characterization and localization of antigens secreted from tachyzoites' cité dans la demande
- JOURNAL OF CELL BIOLOGY vol. 115, 1991, page 5A L. D. SIBLEY ET AL 'Calcium regulated secretion and modification of host-cell endocytic compartments by Toxoplasma'
- PARASITOLOGY RESEARCH vol. 76, 1990, pages 473 - 478 F. DARCY ET AL 'Identification and biochemical characterization of antigens of tachyzoites and bradyzoites of Toxoplasma gondii with cross-reactive epitopes' cité dans la demande
- MOLECULAR AND BIOCHEMICAL PARASITOLOGY vol. 45, 1991, pages 249 - 260 M.A. LERICHE ET AL 'Characterization of the protein contents of rhoptries and dense granules of Toxoplasma gondii tachyzoites by subcellular fractionation and monoclonal antibodies'
- INFECTION AND IMMUNITY. vol. 55, no. 9, Septembre 1987, WASHINGTON US pages 2137 - 2141 L. D. SIBLEY ET AL 'Ultrastructural localization of an intracellular Toxoplasma protein that induces protection in mice' cité dans la demande

## Description

La présente Invention est relative au clonage du gène codant pour un antigène d'excrétion-sécrétion de 28,5 kDa de toxoplasme, et à l'obtention de fragments peptidiques représentant des épitopes de celui-ci, ainsi qu'à des préparations dudit antigène et de ses fragments, et à leurs utilisations.

La toxoplasmose est une des infections à protozoaires les plus répandues, aussi bien chez l'homme que chez l'animal. Elle est responsable d'environ 25% des décès chez les patients atteints du SIDA. L'infection congénitale, cause d'avortements ou de malformations néo-natales sévères chez l'homme et les animaux domestiques, pourrait être prévenue. En effet, on sait que la primo-infection induit une immunité de longue durée.

Dans la recherche d'antigènes protecteurs permettant le développement d'un vaccin contre la toxoplasmose, différents antigènes ont été étudiés. L'équipe des Inventeurs s'est en particulier intéressée aux antigènes d'excrétion-sécrétion (ESA) des tachyzoïtes. Il a en effet été établi, au cours d'expérimentations aussi bien chez l'homme que chez l'animal, que les antigènes ESA étaient immunogènes. Il a également été montré que certains des ESA possèdent des épitopes communs avec des antigènes des bradyzoites. Or, les bradyzoites sont la forme résistante du parasite.

Différentes approches, comprenant en particulier la production d'anticorps monoclonaux, le marquage à l'or colloidal, et la biologie moléculaire, ont conduit l'équipe des Inventeurs à la caractérisation de quatre antigènes communs [CHARIF et al. Exp. Parasitol., 71:117 (1990)], [CESBRON-DELAUW et al., Proc. Natl. Acad. Sci. USA, 86:7537 (1989)], [DARCY et al., Parasitol. Res. 76:478, (1990)]. Le principal, dénommé Gra2 ou GP28.5,est une glycoprotéine de 28.5 kDa, et il a été montré qu'il était un constituant de la matrice des granules denses des tachyzoïtes, et qu'il était associé avec le réseau de micro-villosités de la vacuole parasitophore du parasite, après invasion de l'hôte.

Un antigène de 28 kDa (P28), considéré comme similaire à l'antigène GP28.5, a été décrit par SIBLEY et SHARMA [SIBLEY et al. Infect. Immun. 55:2137 (1987)], et une séquence d'ADN codant pour cet antigène a été publiée par PRINCE et al. [Molec. Biochem. Parasitol. 34:3 (1989)].

La présente Invention s'est fixé pour but l'obtention de préparations purifiées de l'antigène GP28.5, ainsi que l'obtention de cet antigène sous forme recombinante, et sa caractérisation immunologique. En particulier, la présente Invention a pour but la localisation et la caractérisation d'épitopes spécifiques de l'antigène GP28.5.

Les Inventeurs sont parvenus à obtenir une préparation purifiée de l'antigène GP28.5, et ont démontré l'effet protecteur d'une immunisation par cette préparation contre l'infection par *Toxoplasma gondii* chez la souris.

Les Inventeurs ont également cloné l'ensemble du gène codant pour l'antigène GP28.5, et ont localisé les introns et les exons, ainsi que les régions non-codantes en 5' et 3'.

La présente invention a pour objet un fragment d'acide nucléique, caractérisé en ce qu'il comprend une séquence codant pour l'antigène GP28.5 de toxoplasme. Un fragment d'acide nucléique conforme à l'Invention est représenté dans la liste des séquences en annexe sous le numéro SEQ.ID.NO:1. Par : "séquence codant pour l'antigène GP28.5 de toxoplasme", on entend non seulement la séquence codante identifiée dans la séquence SEQ.ID.NO:1, mais également toute séquence qui, compte tenu de la dégénérescence du code génétique, code pour le polypeptide représenté dans la liste des séquences en annexe sous le numéro SEQ.ID.NO:2.

Les Inventeurs ont également montré que l'antigène GP28.5 contient plusieurs épitopes majeurs spécifiques des cellules B ; l'un d'entre eux, qui est reconnu par un anticorps monoclonal de souris dénommé TG17-179 [CHARIF et al., Exp. Parasitol., 71:117 (1990)] est localisé dans la séquence C-terminale de quinze acides aminés de l'antigène GP28.5.

Les Inventeurs ont caractérisé l'épitope défini ci-dessus et ont montré qu'il comprenait au moins les 8 acides aminés C-terminaux de GP 28.5. En outre, ils ont montré que cet épitope est également un épitope majeur reconnu par un certain nombre de sérums de patients atteints d'infections aiguës ou chroniques par *T. gondii*.

L'Invention a donc pour objet un fragment d'acide nucléique codant pour un polypeptide comprenant au moins un épitope de l'antigène GP28.5, éventuellement fusionné à une autre séquence polypeptidique, caractérisé en ce que ledit polypeptide est constitué par les 8 à 15 acides aminés C-terminaux de la séquence SEQ.ID.NO:2.

L'Invention a également pour objet un fragment peptidique constituant un épitope de l'angigène GP28.5, éventuellement fusionné avec une autre séquence polypeptidique, caractérisé en ce que ledit fragment peptidique est constitué par les 8 à 15 acides aminés C-terminaux de la séquence SEQ.ID.NO :2.

L'Invention a également pour objet un procédé de production d'un fragment peptidique tel que défini ci-dessus, lequel procédé est caractérisé en ce qu'il comprend une étape au cours de laquelle l'on procède à la mise en culture de cellules eucaryotes ou procaryotes transformées (et en particulier des microorganismes) par un fragment d'acide nucléique conforme à l'Invention.

Les polypeptides recombinants conformes à l'Invention, exprimés chez *E. coli*, conservent des épitopes majeurs impliqués dans la réponse polyclonale à l'antigène GP28.5, et ce, bien que l'expression chez *E. coli* ne maintienne pas l'intégrité structurale de la protéine GP28.5 qui est une protéine glycosylée.

Si l'on souhaite toutefois obtenir d'autres épitopes tels que des épitopes de nature glucidique, ou des épitopes correspondant à des structures tertiaires et quaternaires, on choisira de produire la GP28.5 recombinante dans des systèmes eucaryotes tels que par exemple, les levures ou les baculovirus.

La séquence des 5 acides aminés C-terminaux de l'antigène GP28.5 est reconnue en immuntransfert par l'anticorps monoclonal de souris TG 17-179. Un ELISA compétitif avec des peptides plus longs a montré que l'immunoréactivité était conservée pour des peptides de 8 résidus ou plus, et perdue quand le peptide était réduit aux 6 derniers résidus C-terminaux ou moins. Des expériences avec l'octapeptide dépourvu du résidu glutamine C-terminal ont montré qu'il était alors 20 fois moins actif. En revanche, ni l'addition de résidus à l'extrémité C-terminale, ni la substitution de la fonction COOH terminale ne changent l'immunoréactivité de l'épitope. En outre, des expériences de compétition entre l'anticorps monoclonal TG 17-179 et des sérums de patients infectés ont montré que l'épitope défini par cet anticorps monoclonal est également un épitope majeur pour les anticorps polyclonaux humains.

L'Invention a également pour objet des compositions antigéniques caractérisées en ce qu'elles comprennent au moins un fragment peptidique conforme à l'Invention.

L'Invention englobe également un procédé de préparation d'anticorps anti-GP28.5, polyclonaux ou monoclonaux, caractérisé en ce qu'il comprend une étape au cours de laquelle on immunise un animal avec une composition antigénique conforme à l'Invention.

Les compositions antigéniques conformes à l'Invention permettent également la préparation de réactifs de diagnostic, ou de vaccins anti-toxoplasmes. L'Invention englobe également ces réactifs et ces vaccins.

La présente Invention sera mieux comprise à l'aide du complément de description qui va suivre, qui se réfère à des exemples concernant le clonage du gène codant pour l'antigène GP28.5 de *Toxoplasma gondii*, et l'identification des épitopes spécifiques de cet antigène.

### I) CLONAGE ET EXPRESSION DU GENE CODANT POUR LA GP28.5 DE T. gondii

Sauf précision contraire, les techniques de manipulation des acides nucléiques et de clonage moléculaire utilisées dans les exemples qui suivent sont celles décrites par SAMBROOK et al.[A Laboratory Manual (second edition), Cold Spring Harbor Laboratory Press, Cold Spring Harbor, NY. (1989)]

L'anticorps TG 17-179 a été obtenu à partir de souris BALB/C immunisées avec des antigènes ESAs comme décrit par CHARIF et al. [Exp. Parasitol. 71:114 (1990)].

### EXEMPLE 1 : CLONAGE DU GENE CODANT POUR LA GP28.5 de T. gondii

1°) Obtention des toxoplasmes.
   Des tachyzoïtes ont été obtenus à partir du fluide péritonéal de souris infectées trois jours auparavant avec la souche RH de *Toxoplasma gondii.* Les parasites ont été récoltés dans du milieu RPMI 1640 (GIBCO BRL), filtrés à travers des membranes de polycarbonate (diamètre de pores 3 microns) (NUCLEPORE.) et lavés deux fois dans le même milieu.
2°) Préparation d'acide nucléique de Toxo*plasma gondii*. L'ARN total a été isolé de tachyzoites de *Toxoplama gondii* de la souche RH par extraction au lithium/urée, et l'ARN poly (A+) a été purifié par passage sur une colonne d'oligo (DT)-cellulose.
3°) Construction de la banque d'ADNc de tachyzoites.
   a) Construction dans λgt11
      La construction de la banque d'ADNc de toxoplasme dans le phage lambda gt11 a été décrite par CESBRON-DELAUW et al. [CESBRON-DELAUW et àl. Proc. Natl. acad. Sci. USA, 86:7537 (1989)]. La banque, après amplification, a été étalée sur des cellules de *E. coli* Y1090 et criblée en utilisant l'anticorps monoclonal TG 17-179. Les clones positifs ont été détectés par incubation avec des anticorps anti IgG de souris conjugués à la peroxydase, suivis d'un marquage au 4-chloro-1 naphtol.
      Trois clones de λgt11 ont été sélectionnés : il s'agit des clones FM3, FM1, et FM16, contenant respectivement des inserts de 450, 550, et 650 paires de base. Des fragments de restriction EcoRI de ces clones ont été sous-clonés dans les vecteurs M13, mp18, et mp19, et séquencés par la méthode de SANGER [FEINBERG et al. Anal. Biochm. 137:266 (1984)].
      Le séquençage a montré que ces trois clones codent tous pour l'extrémité C-terminale de GP28.5.
   b) Construction dans λZapII

La banque d'ADNc de tachyzoites a été construite dans le vecteur λZapII, en utilisant le "ZAP cDNA SYNTHESIS KIT" (STRATAGENE). L'ADNc a été synthétisé en utilisant comme matrice 5 µg d'ARN poly-A des tachyzoites, à l'aide de la transcriptase inverse (MMLVRT) pour la synthèse du premier brin, et de l'ADN polymérase I et la RNase H pour la synthèse du second brin. Après ligature en 3' d'un adaptateur EcoRI, et après digestion en 5' par XhoI, l'excès d'adaptateur a été éliminé par chromatographie sur acrylamide-agarose (Ac-A 34, IBF). Les ADNc ont ensuite été ligaturés dans le gène LacZ de lZapII ("UNI ZAP XR", STRATAGENE) et encapsidés in vitro ("GIGAPAC II GOLD PACKAGING EXTRACT", STRATAGENE). Avant amplification, la librairie comprend 10⁶ phages recombinants.

Après amplification, les phages ont été étalés sur E. coli XLI-Blue et la banque a été criblée avec d'une part, le sérum polyclonal de souris dirigé contre l'antigène GP28.5 purifié (dilution au 1/100) et, d'autre part, l'anticorps monoclonal TG17-179 (dilution au 1/500), tous deux dilués dans du tampon TBS (10 mM TRIS HCl pH 8, 15mM NaCl). La co-infection des bactéries par λZapII et un phage auxilliaire (R408, Stratagène) a permis d'exciser le phagemide pBluescript contenant les inserts d'ADNc clonés. L'ADN simple brin obtenu à partir du phagemide en présence du même phage auxilliaire a été directement employé pour séquencer les inserts d'ADNc par la méthode des di-déoxynucléotides.

Le criblage de la banque d'ADNc construite dans le vecteur d'expression λZapII a permis l'obtention d'ADNc plus longs. Le plus long de ceux-ci, dénommé "LcDNA" comprend 1100 pb ; sa séquence en 3' est homologue à celle de la P28 de PRINCE et al., mais est plus courte d'au moins 121 bp en 5'.
4°) Construction d'une banque génomique de *Toxoplasma gondii* dans le phage EMBL 3 :
   Après digestion partielle de l'ADN génomique des tachyzoites avec MboI, la banque génomique a été construite comme décrit par CESBRON-DELAUW et al. [Proc. Natl. Acad. Sci., USA, 86:7537 (1989)].

La banque construite dans le phage EMBL3 a été étalée sur E. coli P2392, et criblée à l'aide d'un fragment de restriction de l'ADNc (fragment EcoR1-SalI de 195 pb), utilisé comme sonde, après marquage préalable au ³²P. Les hybridations ont été effectuées à 65 °C, dans du tampon 5X DENHARDT [le tampon 50X DENHARDT contient 1% de SAB, 1% de PVP, 1% de Ficoll (w/v)] et 6,6X SSC (10X SSC comprend 3M NaCl, 0,3M NA₃C₆H₅O₇, 2 H₂O, pH 7,2).

Un clone génomique dénommé Gra2-EMBL3 a été sélectionné ; à partir de ce clone, un fragment de 2040 pb, comprenant l'ensemble du gène ainsi que 780 pb de séquence flanquante 5' et 32 pb de séquence flanquante 3', a été sous-cloné.

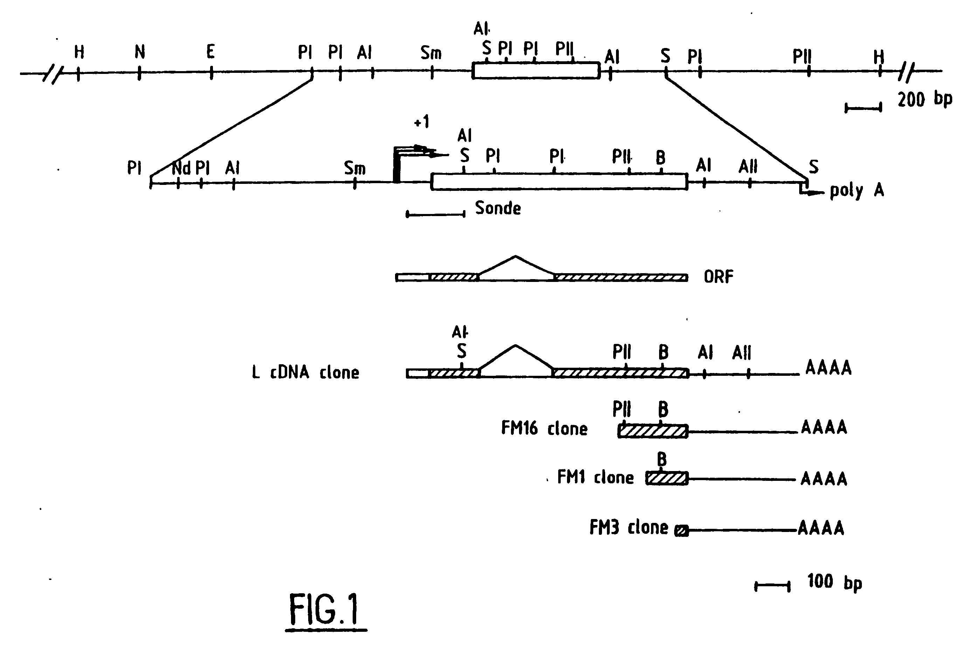

La figure 1 représente la carte de restriction partielle sur 5 kb du clone génomique Gra2-EMBL3, ainsi que la carte de restriction partielle d'une région de 2040 pb de celui-ci, qui comprend la totalité du gène GP28.5. Cette figure montre également la région transcrite et la région codant pour la protéine GP28.5. L'analyse en Southern blot d'ADN génomique de tachyzoite hybridé avec une sonde d'ADNc de 1100 pb (clone LcDNA) confirme cette carte de restriction. Elle indique également que le gène codant pour GP28.5 n'est probablement pas répété dans le génome des toxoplasmes.

La ligne du haut est une carte de restriction partielle du clone génomique Gra2-EMBL3 contenant le gène GP28.5 (ce gène est indiqué par une boîte). Le reste de la figure est un agrandissement d'une région de 2040 pb qui représente un sous-clone du clone génomique GP28.5, et qui contient le gène codant pour GP28.5 ainsi que les séquences flanquantes de 780 pb en 5' et de 32 pb en 3'. Le cadre ouvert de lecture de GP28.5 est indiqué. Le fragment EcoRI-SalI de 195 paires de base qui a été utilisé comme sonde pour le criblage des banques est indiqué sous les cartes de restriction ainsi que les clones de cDNA:LcDNA, FM16, FMI, FM3. La séquence AAAA indique la queue poly-A des transcrits. Les régions codantes sont hachurées. Les sites de restriction sont indiqués par les abréviations suivantes : AI = AccI, AII = AvaII ; B = BglI ; E = EcoRV ; H = HindIII ; N = NaeI ; ND = NdeI ; PI = PstI ; PII = PvuII ; S = SalI ; SM = SmaI.

La séquence complète du gène GP28.5 et des régions 5' et 3' non codantes est montrée dans la liste des séquences en annexe sous le numéro SEQ.ID.NO:1. Pour déterminer l'organisation du gène GP28.5, la séquence de la banque génomique a été comparée avec la séquence du clone LcDNA.

Cette comparaison montre que le gène GP28.5 est constitué de deux exons (exon 5':251 pb ; exon 3':800 pb) séparés par un intron de 239 pb.

Un codon ATG initiateur de traduction est présent à la position 886, et le cadre ouvert de lecture se termine à la position 1682 avec un codon TAA. Ce cadre ouvert de lecture code potentiellement pour une protéine de 185 acides aminés dont le poids moléculaire théorique est de 19,8 KDa. Un site potentiel de clivage d'une séquence signal est situé entre les alanines 23 et 24. Le cadre ouvert de lecture est bordé en 3' par une séquence non codante de 32 pb. Un transfert de Northern réalisé sur l'ARN total de tachyzoïtes révèle une seule population d'ARNm codant pour la protéine GP28.5. La taille du messager est estimée approximativement à 1100 pb, ce qui correspond à la taille du clone LcDNA.

Pour définir plus précisément le site d'initiation de la transcription, une extension d'amorces a été effectuée avec l'ARN total de tachyzoites, en utilisant comme amorce un oligo-nucléotide (CM10), correspondant aux positions 888 à 867 de la séquence. Par cette méthode, trois sites potentiels différents d'initiation de la transcription ont été localisés, respectivement à 105, 103 et 102 pb en amont du signal d'initiation de la traduction. Les deux premiers peuvent correspondre à des sites de transcription mineurs alors que le troisième est sans doute le site majeur d'initiation de la transcription.

La séquence d'acides aminés de GP28.5 est plus courte de 67 acides aminés à son extrémité N-terminale que la séquence supposée de l'antigène P28 précédemment publiée par PRINCE et al. Pour vérifier le fait que la séquence SEQ.ID.NO:1 est bien celle qui code pour l'antigène GP28.5, la séquence en acides aminés correspondante a été comparée à celle de cinq peptides résultants du clivage par la trypsine de l'antigène GP28.5 purifié par HPLC comme décrit ci-dessous. La séquence de ces cinq peptides a été entièrement retrouvée dans la séquence en acides aminés déduite de celle du cadre ouvert de lecture du gène cloné.

### EXEMPLE 2 : PREPARATION DE PROTEINES RECOMBINANTES COMPRENANT DES FRAGMENTS DE LA SEOUENCE DE GP28.5 :

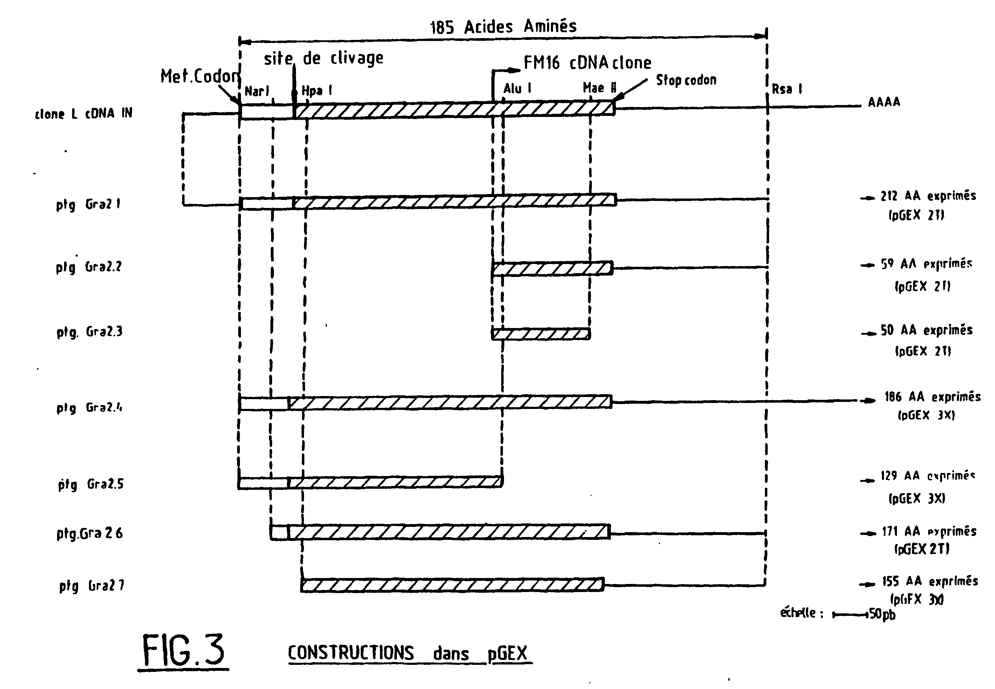

1) Protéines de fusion avec la Glutathion-S-Transférase
   - Deux clones d'ADNc représentant des fragments de la séquence codent pourla protéine GP28.5 ont été sous-clonés dans le plasmide pGEX-2T [SMITH et al. Gene, 67, 31-40 (1988)]. Le premier clone, FM16, code pour les cinquante-neuf acides aminés C-terminaux de GP28.5, et le deuxième clone, L représente 212 acides aminés de GP28.5.
      5 autres fragments (figure 3) codant pour des portions de la protéine GP28.5 ont également été sous-clonés dans les plasmides pGEX-2T et pGEX-3X (SMITH et al., cité) :
   - le premier clone, ptg.Gra2.1, dérivé du clone LcDNA, code pour 212 acides aminés dont les 185 de GP28.5 ;
   - le second clone, ptg.Gra2.2, dérivé du clone cDNA FM16, code pour les 59 acides aminés C-terminaux de la protéine GP28.5 ;
   - le troisième clone, ptg.Gra2.3, code pour 50 acides aminés (acide aminé 127 à acide aminé 176 inclus, voir figure 3) ;
   - le quatrième clone, ptg.Gra2.4, code pour 186 acides aminés ;
   - le cinquième clone, ptg.Gra2.5, code pour 129 acides aminés (partie N-terminale de la protéine GP28.5, jusqu'à l'acide aminé 129 inclus) ;
   - le sixième clone, ptg.Gra2.6, code pour 171 acides aminés (à partir de l'acide aminé 15 inclus) ;
   - le septième clone, ptg.Gra2.7, code pour 155 acides aminés (à partir de l'acide aminé 31 inclus).
      Les plasmides pGEX-2T et pGEX-3X contenant les inserts d'ADN ont été utilisés pour transformer des bactéries E. coli JM 109. L'expression et la purification des protéines recombinantes ainsi que de la GST seule ont été effectuées selon le protocole décrit par SMITH et JOHNSON (référence précitée). Une culture de E. coli JM 109 au milieu de la phase logarithmique a été stimulée par 0,1 mM de IPTG. Après 1 heure de croissance à 37°, les cellules sont refroidies sur la glace et centrifugées à 4000 rpm pendant 15 minutes. Le culot est re-suspendu dans 0,02 M PBS, 0,5 mM PMSF, 1 mM EDTA, 1% TRITON X 100. Les cellules sont soniquées sur la glace et centrifugées à 10 000 g pendant 5 minutes. Les protéines recombinantes sont purifiées à partir du surnageant, par chromatographie d'affinité sur de l'agarose-glutathion. L'expression des protéines recombinantes a été contrôlée par immunotransfert en utilisant l'anticorps monoclonal TG17-179 ou le sérum polyclonal de souris dirigé contre l'antigène GP28.5 purifié. Les protéines recombinantes correspondant aux clones ptg.Gra2.2 (59 acides aminés) et ptg.Gra2.3 (50 acides aminés) ont été purifiées avec un rendement élevé. En revanche, les protéines recombinantes correspondant aux clones ptg.Gra2.1 (212 acides aminés), ptg.Gra2.4 (186 acides aminés) et ptg.Gra2.5 (129 acides aminés) n'ont été obtenus qu'avec des rendements faibles qui semblent dûs à la dégradation des protéines d'une part, et d'autre part à la présence de la séquence signal (les 23 premiers acides aminés de la protéine GP28.5) dans les protéines recombinantes.
2) Protéines de fusion avec la β-galactosidase
   Des phages lysogènes recombinants ont été produits à partir du clone original de λgt11 FM16, chez *E*. *coli* Y1089, selon le procédé décrit par HUYNH et al. [Glover, D.M. ed. vol. 1, 49-78 IRL Press, Oxford (1985)]. Des lysats bruts contenant la β-galactosidase de type sauvage ou bien la protéine de fusion [GP 28.5/β-galactosidase] ont été préparés par induction de cultures en phase logarithmique, par chauffage à 45°C pendant 20 minutes et addition de isopropyl -β-D-thiogalacto-pyranoside à une concentration de 10 mM. Après une incubation supplémentaire à 37°C pendant 1 heure, la culture a été concentrée, dans un tampon 0,01 M Tris, pH8, 0.1 M EDTA, 10 mM NaCl, puis lysée par addition de 0,25 mg de lysozyme, 0,01 M MgCl₂, et 0,2% TRITON X100, et enfin traitée par la DNAseI à 37°C pendant 30 minutes.

Les clones FM1 et FM16 réagissent beaucoup plus fortement avec l'anticorps TG 17-179 que le clone FM3. La carte de restriction montre que les inserts EcoRI de 450 pb (FM3), 550 pb (FM1) et 650 pb (FM16) contiennent des séquences qui se chevauchent.

Le séquençage de ces clones a révélé que les cadres de lecture en phase avec celui de la β-galactosidase étaient de seulement 17 pb pour FM3, et 95 pb et 172 pb pour FM1 et FM16 respectivement, le reste des inserts correspondant à la région 3' non traduite plus la queue polyA. Il apparaît donc que le peptide correspondant aux 5 amino acides C-terminaux de l'antigène GP28.5 codé par le clone FM3, est suffisant pour obtenir une réaction antigénique.

### II) PURIFICATION DE L'ANTIGENE GP28.5

### EXEMPLE 3 : PURIFICATION DE L'ANTIGENE GP28.5 PAR HPLC

10¹⁰ tachyzoltes de la souche RH ont été lavés deux fois avec 10 mM PBS, pH 7,2. Après centrifugation à 1000 g pendant 10 minutes, le culot a été incubé toute la nuit à 4°C sous agitation douce, à une concentration de 10⁹ parasites/ml dans du tampon TEN (10 mM TRIS-HCL, pH 7,4 ; 2 mM EDTA ; 150 mM NaCl) additionné de 1% de NONIDET P40, de 100 U/ml d'aprotinine et de 40 µM de PMSF. Après centrifugation à 3500 g pendant 20 minutes, le surnageant a été récupéré et filtré sur une membrane MILLIPORE de 0.22 µm. Les protéines de tachyzoites extraites au NP40 ont été purifiées par chromatographie HPLC en phase inverse sur une colonne VYDAC C18 (300 Å, taille des particules : 7 µm, dimensions de la colonne : 500x9mm). 8 ml d'extrait de protéines brutes ont été chargés sur la colonne C18 à 0% de solvant B [le solvant A est constitué de 0,5% v/v de TFA dans l'eau ; Le solvant B est un mélange 25/75/0,45 (v/v) d'eau/acétonitrile/TFA]. Après 10 minutes d'élution isocratique, les protéines sont éluées avec un gradient de 0 à 100% de solvant B, pendant une durée de 180 minutes, à une vitesse d'élution de 2 ml par minute, et détectées à 215 nanomètres. Pour localiser l'antigène GP28.5, chaque fraction HPLC a été lyophilisée, et le lyophilisat a été dilué dans 100 µl d'un mélange TEN/PBS, [10 mM, pH 8 (v/v)]. 1 µl de chaque fraction a été testé par dot-blot en utilisant l'anticorps monoclonal TG17-179. 10 µl (ce qui correspond à environ 12 µg de protéine purifiée) de chacune des fractions qui ont montré une réaction positive, ont été déposés sur gel d'acrylamide en présence de 2-ME, Une coloration du gel au nitrate d'argent a permis de vérifier leur homogénéité et leur pureté. Un gel similaire a été réalisé et utilisé pour le transfert électrophorétique ,sur membrane de nitro-cellulose. L'antigénicité des fractions a été évaluée en incubant la membrane avec l'anticorps monoclonal TG17-179. La détection a été faite avec des anticorps anti-IgG de souris, marqués à la peroxydase.

Le composant majeur de l'éluat est la protéine GP28.5. Le seul contaminant décelé est une protéine de 65 kDa. qui est également reconnue par l'anticorps TG17-179, et qui peut donc représenter soit un dimère de GP28.5, soit un antigène apparenté.

### III) PROPRIETES IMMUNOLOGIOUES DE L'ANTIGENE GP28.5 ET DE SES FRAGMENTS

### EXEMPLE 4 : IMMUNISATION DES SOURIS ET ESSAIS DE PROTECTION.

Des souris femelles OF1 âgées de 8 à 10 semaines ont été immunisées de la façon suivante : quarante-deux, trente-cinq, vingt-sept et sept jours avant l'infection, les antigènes suivants :
- ESA correspondant à la sécrétion de 5.10⁷ tachyzoïtes ; ou bien,
- 15 µg de GP28.5 purifiée par HPLC comme décrit à l'exemple 3 ont été mis en suspension dans 100 µl de PBS, émulsifié avec 100 µl d'adjuvant incomplet de FREUND et administrés par injection sous-cutanée.

Des souris témoin ont été traitées selon le même protocole par du PBS additionné de 100 µl d'adjuvant incomplet de FREUND.

Le jour de l'infection, les sérums ont été collectés, puis une suspension de 1200 kystes dans 0,3 ml de PBS, pH 7,2 a été administrée oralement.

Les sérums des souris immunisées avec les ESA ou la GP28.5 purifiée par HPLC ont été testés par transfert immuno-électrophorétique, contre des antigènes ESA de tachyzoites extraits au NP40, afin de déterminer s'ils reconnnaissent l'antigène GP28.5.

Il apparaît que le profil d'immuno-réactivité est sensiblement le même que la révélation soit faite avec l'immunsérum de souris ou bien avec l'anticorps monoclonal TG17-179 : dans les deux cas, trois bandes d'environ 100, 65 et 28 KDa sont révélées. Les deux bandes de poids moléculaire le plus élevé qui sont reconnues par l'anticorps TG17-179 représentent probablement des polymères de GP28.5

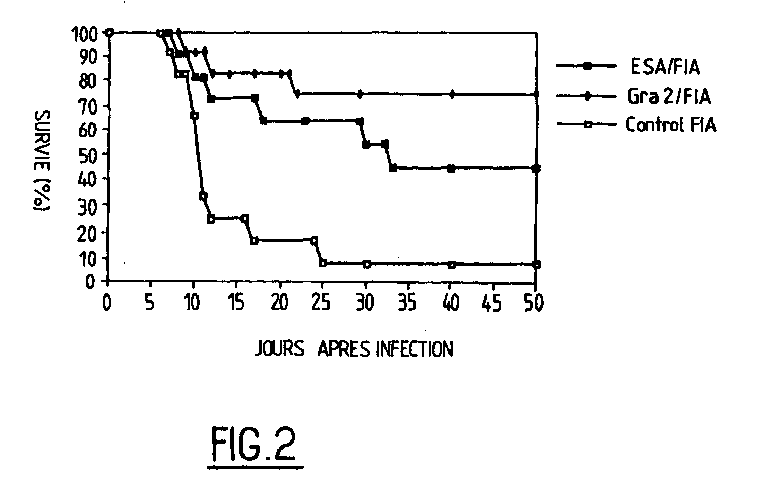

La figure 2 représente le pourcentage de souris survivantes après l'infection. Cette figure montre que, alors que seulement 25% des souris témoin immunisées uniquement avec l'adjuvant incomplet de FREUND survivent 15 jours après l'infection, 75% des souris immunisées avec l'antigène GP28.5 purifié par HPLC sont encore vivantes 50 jours après l'infection. Lorsque les souris sont immunisées avec les antigènes ESA, 45% survivent 50 jours après l'infection. Ces résultats montrent que l'immunisation avec l'antigène GP28.5 purifié par HPLC conduit à une protection significative.

### EXEMPLE 5 : REACTIVITE DES SERUMS HUMAINS AVEC LES PROTEINES RECOMBINANTES ET RECONNAISSANCE DE L'EPITOPE C-TERMINAL

Les sérums humains ont été obtenus à partir de patients porteurs d'une infection chronique par Toxo*plasma gondii* (TG positif). Des sérums de patients sains ont été utilisés comme témoin (TG négatif). Le test a été réalisé par ELISA, et la révélation faite en utilisant des anticorps anti-IgG humaines biotinylées ; la fixation de ces anticorps a été décelée en utilisant des complexes streptavidine-biotine marqués à la peroxydase. Le substrat de peroxydase utilisé est l'OPD et la densité optique a été mesurée à 492 nm et exprimée par rapport à un témoin réalisé en utilisant comme antigène la GST, selon la formule suivante :
DO = DO du sérum réagissant avec la protéine recombinante de 59 acides aminés - DO du sérum réagissant avec la GST. Dans ces conditions, la moyenne de la densité optique des sérums TG-négatifs était de 0,23. 74% des cinquante-neuf sérums TG-positifs étaient au dessus de cette densité optique. La corrélation avec un test conventionnel des anticorps anti-TG est de : R = 0,74 P.

Malgré cette corrélation, des patients possédant de 50 à 100 UI/ml d'IgG anti-TG montrent des valeurs très dispersées de niveaux d'anticorps contre la protéine recombinante FM16 (les densités optiques s'échelonnent entre 0,35 et 1,5). Il semble donc qu'un niveau homogène d'anticorps anti-TG puisse en fait réfléter des réponses variées à l'antigène GP28.5.

Dix sérums négatifs en ELISA contre la protéine contenant les cinquante-neuf acides aminés ont été testés en immunotransfert contre la protéine de fusion contenant les 212 acides aminés (clone LcDNA). Huit de ces sérums réagissent avec la protéine comprenant les 212 acides aminés, alors que 7 des sérums de contrôle réagissent négativement. Il est probable que ces 8 sérums reconnaissent des épitopes présents sur la protéine de 212 acides aminés, et plus proches de l'extrémité N-terminale que ceux portés par la protéine comprenant les cinquante-neuf acides aminés.

La fixation de TG 17-179 à l'antigène recombinant FM16 a été testée en ELISA compétitif en présence de sérums de patients infectés ; le sérum a été ajouté à une dilution de 1/100 à la solution de l'anticorps monoclonal.

Ces essais de compétition entre TG 17-179 et des sérums de patients infectés montrent que les anticorps polyclonaux humains réagissent avec l'épitope C-terminal de la GP28.5 : parmi les 12 sérums humains testés, 10 inhibent à des degrés variés la fixation de TG 17-179 à la protéine de fusion FM16.

La réactivité des protéines de fusion produites par les clones FM1 et FM3 avec l'anticorps TG 17-179 a également été testée ; FM1 et FM16 réagissent beaucoup plus fortement avec l'anticorps TG 17-179 que le clone FM3.

Or, le séquençage de ces clones a révélé que le cadre de lecture en phase avec celui de la β-galactosidase, est de seulement 17 pb pour FM3, et 95 pb et 172 pb pour FM1 et FM16 respectivement. Il apparaît donc que le peptide codé par le clone FM3, et correspondant aux 5 amino acides C-terminaux de l'antigène GP28.5 est suffisant pour obtenir une réaction antigénique.

Cependant, dans la mesure où il est également apparu que la liaison de l'anticorps monoclonal TG 17-179 à ce peptide FM3 est plus faible que la liaison dudit anticorps aux peptides codés par les clones plus longs FM1 et FM16, les Inventeurs ont entrepris de déterminer la longueur optimale de l'épitope.

### EXEMPLE 6 : IMMUNOREACTIVITE DE PEPTIDES SYNTHETIOUES CORRESPONDANT A L'EXTREMITE C-TERMINALE DE GP28.5

Des peptides se chevauchant et couvrant la séquence carboxy-terminale de GP28.5 (1 à 15 résidus) ont été synthétisés, en utilisant la méthode de MERRIFIELD.

Ils ont été testés par ELISA compétitif avec la protéine de fusion FM16.

Les quinze acides aminés terminaux de GP28.5 contiennent l'épitope réagissant avec l'anticorps monoclonal TG17-179. La capacité de ce peptide à inhiber la réactivité en ELISA contre la protéine recombinante contenant les cinquante-neuf acides aminés, de sérums obtenus à partir de patients atteints d'infections aigues ou chroniques a été testée. Les deux types de sérums (aigu et chronique) sont inhibés, à un degré varié, par le peptide C-Terminal de quinze acides aminés. Pour les sérums chroniques, le pourcentage d'inhibition varie de 8 à 100% et on n'observe aucune corrélation avec la densité optique de départ. Le faible taux d'inhibition observé pour certains sérums peut être dû à la présence d'anticorps à faible affinité pour ce peptide. Toutefois, même en multipliant par dix la concentration du peptide, le pourcentage d'inhibition n'est pas augmenté. La spécificité de l'inhibition a été démontrée en utilisant comme témoin un peptide de séquence différente et de longueur similaire. Le pourcentage d'inhibition des sérums aigus varie de 15 à 90% et est également indépendant de la densité optique de départ.

Les études d'inhibition réalisées montrent donc que le peptide de 15 acides aminés comprend un l'épitope majeur de la région C-Terminale de cinquante-neuf acides aminés, pour quatre sur douze des sérums obtenus à partir de patients chroniques, et pour trois sur douze des sérums obtenus à partir de patients atteints d'infections aigues. En outre, trois sur douze des sérums chroniques, et quatre sur douze des sérums aigus, montrent une inhibition partielle (35-80% d'inhibition).

Il apparaît donc que la réponse polyclonale à l'antigène GP28.5 implique une réactivité avec les quinze acides aminés C-Terminaux, réactivité dont le degré varie suivant les individus. En revanche, le degré de cette réponse ne varie apparemment pas entre l'infection aigue et l'infection chronique, aucune différence dans le pourcentage d'inhibition moyen pour chacun des deux groupes n'ayant été observée.

Toutefois, cinq sur douze des sérums chroniques et cinq sur douze des sérums aigus qui réagissent avec la région C-Terminale de cinquante-neuf acides aminés de GP28.5 ne réagissent pas avec le peptide de quinze acides aminés C-terminaux, ce qui montre que d'autres épitopes B majeurs existent dans cette région de cinquante-neuf acides aminés et notamment dans le fragment correspondant aux amino-acides 127-176 de la séquence de l'antigène GP28.5.

Dans le cas de peptides C-terminaux présentant moins de 15 acides aminés, les résultats sont les suivants :
L'inhibition la plus forte de la liaison de l'anticorps monoclonal TG 17-179 à la protéine de fusion est obtenue avec des peptides comprenant entre 11 et 15 résidus.
L'inhibition est toutefois obtenue avec l'octapeptide comprenant les 8 résidus C-terminaux.
La liaison de TG 17-179 à l'heptapeptide C-terminal est 8 fois moins importante que sa liaison à l'octapeptide. Enfin l'immunoréactivité du peptide synthétique est perdue quand il comprend 6 acides aminés C-terminaux ou moins.
L'octapeptide dépourvu de sa fonction COOH et l'octapeptide comprenant deux résidus C-terminaux additionnels (Alanine) ont été également été testés. Ces deux peptides ont pratiquement la même activité que l'octapeptide C-terminal : ceci montre que la fonction carboxyle n'est pas nécessaire à l'immunoréactivité de l'épitope reconnu par TG 17-179.
Des mêmes peptides ont été testés en ELISA direct. Dans ces conditions l'immunoréactivité en fonction de la longueur du peptide décroît plus rapidement : elle en effet perdue pour des peptides de longueur inférieure à 10 résidus. En conséquence, il apparaît qu'en ELISA direct, l'anticorps monoclonal nécessite un peptide plus long que dans le test par inhibition. Dans ce cas particulier, il est peu probable que ceci soit dû à une mauvaise absorption des peptides les plus courts sur la plaque de microtitration, étant donné la nature hydrophobique des amino-acides desdits peptides.

Des peptides dépourvus de résidus C-terminaux ont également été synthétisés : alors que l'incubation de TG 17-179 avec l'octapeptide synthétique couvrant l'ensemble des résidus C-terminaux inhibe la liaison de la protéine de fusion recombinante, l'heptapeptide correspondant dépourvu du résidu glutamine carboxy-terminal est 64 fois moins actif en tant que compétiteur, et l'hexapeptide dépourvu des deux résidus carboxy-terminaux est 104 fois moins actif.

### LISTE DE SEQUENCES :

NOMBRE DE SEQUENCES: 2
1) INFORMATION POUR LA SEQ ID NO: 1:
   (i) CARACTERISTIQUES DE LA SEQUENCE:
      (A) LONGUEUR: 2152 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Toxoplasma gondii
   (ix) CARACTERISTIQUE ADDITIONELLE:
      (A) NOM/CLE: CDS
      (B) EMPLACEMENT: join(886..1035, 1275..1682)
   (ix) CARACTERISTIQUE ADDITIONELLE:
      (A) NOM/CLE: exon
      (B) EMPLACEMENT: 886..1035
   (ix) CARACTERISTIQUE ADDITIONELLE:
      (A) NOM/CLE: exon
      (B) EMPLACEMENT: 1275..1682
   (ix) CARACTERISTIQUE ADDITIONELLE:
      (A) NOM/CLE: intron
      (B) EMPLACEMENT: 1036..1274
   (xi) DESCRIPTION DE LA SEQUENCE: SEQ ID NO: 1:
   CTGCAGTCTT AATCATTTCC ACATAGTTTT TGTTCCCCCA GACAATCAAT CCTGGCTGAG     60
   CCCCCCATGT ACGCGTTACC ACCTGCCAGT GCATATGGGT TTGCATATTT TTGCTGAAGT    120
   CCGAAAGAGG GGCCACGCAA AACGTTACCG GTTTGCTGCA GGCAGCCAGG TAGGTGGAAC    180
   AGATCTCCAG CGAGTACGAC CACTGTGCGT GTACTTACGC CAAAAGGAAA ATACACCTGC    240
   ACCGCTATTA AGCGGCAGTC GTCTACCTGA ATCGTCTCCC CGGCCTCATC ATTTTGTTCG    300
   ACACAAGTTT CCATTAGGAC TTTGTGACAG TCGTCTGCTC TCGACCTTCC AACCGTCTCG    360
   TGGACGAAAA ATCCTCGGGT GACTTGCCGT GGACGAACGC CTCCCGTTTG CTTCTACAAG    420
   TGACTATGCG ATAGGTTCCG CAGTGCAGCC AGGCTTGCGA AAAACAAGTT CGTCGCAAAA    480
   GGTTAATTAC CTACGCACCA CGAAGGAAAA CGCGTATCAC GTCAGTCCTT ACGGTCAATA    540
   TACAAATACT TGGCCGTCCA GTGGAGGCAA CGTGCCCGTC GCACGGTGAT ACTGACTGGT    600
   GACTTGCACG TACGTCTCTC GCCGGCGTCC AAACCAAATT GACCCGGGGC AGCCTACTCC     660
   CTGTCGTCCC TTAGGCTAAG TGCGAGCAAC ATCTCTACAC AGAGACGACG CCAGAGACGC     720
   AAAATGAACA GCGGAACCTG CGTCGCTGTC TGTCCTGCGA ACTGATGACA GAAAGGGTCA     780
   TTAAACGATT TCTTTTGCAA TTCGCGTCGT TATCGCACGT TGTTTCTCTT CCCACGAATA     840
   GTTGTTTTGA TTAGATATTG CTTCTTCTCC ACATATCGCC TCACA ATG TTC GCC         894
                                                    Met Phe Ala
                                                      1
   GTA AAA CAT TGT TTG CTG GTT GTT GCC GTT GGC GCC CTG GTC AAC GTC       942
   Val Lys His Cys Leu Leu Val Val Ala Val Gly Ala Leu Val Asn Val
        5                  10                  15
   TCG GTG AGG GCT GCC GAG TTT TCC GGA GTT GTT AAC CAG GGA CCA GTC       990
   Ser Val Arg Ala Ala Glu Phe Ser Gly Val Val Asn Gln Gly Pro Val
    20                  25                  30                  35
   GAC GTG CCT TTC AGC GGT AAA CCT CTT GAT GAG AGA GCA GTT GGG          1035
   Asp Val Pro Phe Ser Gly Lys Pro Leu Asp Glu Arg Ala Val Gly
                    40                  45                  50
   TAAGTTGGCA AAAGTAATGA TAGAGGCAGG GGTTGAACGA TAGGCGGCTG CAGATTTGTA    1095
   TAACACAACA TGATGTAGCT GCCACGGTTT TTTTTCGGAG AGTGATGCCG TCTGACTGTC    1155
   ATCGCACCCA TGGGAGCTAG GGAGGTGCGC TTCTGTGTGA TATGTATTGT CCTAGTCCAA    1215
   TTTCCCACGC ACTGTAGTGT CTTGAGACTC GGTGCCATGT AGAATTTTGT GTCTGCAGA     1274
   GGA AAA GGT GAA CAT ACA CCA CCA CTC CCA GAC GAG AGG CAA CAA GAG      1322
   Gly Lys Gly Glu His Thr Pro Pro Leu Pro Asp Glu Arg Gln Gln Glu
                    55                  60                  65
   CCA GAA GAA CCG GTT TCC CAA CGT GCA TCC AGA GTG GCA GAA CAA CTG      1370
   Pro Glu Glu Pro Val Ser Gln Arg Ala Ser Arg Val Ala Glu Gln Leu
                70                  75                  80
   TTT CGC AAG TTC TTG AAG TTC GCT GAA AAC GTC GGA CAT CAC AGT GAG      1418
   Phe Arg Lys Phe Leu Lys Phe Ala Glu Asn Val Gly His His Ser Glu
            85                  90                  95
   AAG GCC TTC AAA AAA GCA AAG GTG GTG GCA GAA AAA GGC TTC ACC GCG      1466
   Lys Ala Phe Lys Lys Ala Lys Val Val Ala Glu Lys Gly Phe Thr Ala
       100                 105                 110
   GCA AAA ACG CAC ACG GTT AGG GGT TTC AAG GTG GCC AAA GAA GCA GCT      1514
   Ala Lys Thr His Thr Val Arg Gly Phe Lys Val Ala Lys Glu Ala Ala
   115                 120                 125                 130
   GGA AGG GGC ATG GTG ACC GTT GGC AAG AAA CTC GCG AAT GTG GAG AGT      1562
   Gly Arg Gly Met Val Thr Val Gly Lys Lys Leu Ala Asn Val Glu Ser
                   135                 140                 145
   GAC AGA AGC ACT ACG ACA ACG CAG GCC CCC GAC AGC CCT AAT GGC CTG      1610
   Asp Arg Ser Thr Thr Thr Thr Gln Ala Pro Asp Ser Pro Asn Gly Leu
               150                 155                 160
   GCA GAA ACC GAG GTT CCA GTG GAG CCC CAA CAG CGG GCC GCA CAC GTG      1658
   Ala Glu Thr Glu Val Pro Val Glu Pro Gln Gln Arg Ala Ala His Val
           165                 170                 175
   CCC GTC CCA GAC TTT TCG CAG TAATGTTGAC TACGACGAAA GTGATGCGCA         1709
   Pro Val Pro Asp Phe Ser Gln
       180                 185
   GGCTGGAAAG CCGCTGAAGG GAGAAGTCTA CAAAGCCGAT CAGTGAAAAA TGTGTGGGGA    1769
   GGTGGTCTTG TTGCAGGAAT GCAATGTGTT AAGCATCGTG TTCGAATGCA GTGCGTGTAT    1829
   CAGTTGTGCG CGGAAGGACA CTGCTTCAAT GTTAAGAACC TGTTTTCTCC GTAGAGAGGA    1889
   CCAAAAGACG ATTGCAAAAC TGGTATGTAC GCAATAGCCC AATGCCGGAC GTCAGTTGGT    1949
   TGTATGTGAC GCTCCCAGAT GTCATATGCC TTGTGAGTGT GTCTGGGATG CAAGTTTTTG    2009
   GTGTGCGTTG ATTTCGCCAG CTTATGACAG TGGCAGACGA ATTATTGACA TGATACAAGG    2069
   ACGCAGAAAG GAACAAACAC CGTAGTTCCA GTCGACACAG AAAGGGAGGG TAAAGAAAGT    2129
   AATTGAAAGG TGATTTTAGA TAA                                            2152
(2) INFORMATION POUR LA SEQ ID NO: 2:
   (i) CARACTERISTIQUES DE LA SEQUENCE:
      (A) LONGUEUR: 185 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Phe Ala Val Lys His Cys Leu Leu Val Val Ala Val Gly Ala Leu
   1 5 10 15
   Val Asn Val Ser Val Arg Ala Ala Glu Phe Ser Gly Val Val Asn Gln
   20 25 30
   Gly Pro Val Asp Val Pro Phe Ser Gly Lys Pro Leu Asp Glu Arg Ala
   35 40 45
   Val Gly Gly Lys Gly Glu His Thr Pro Pro Leu Pro Asp Glu Arg Gln
   50 55 60
   Gln Glu Pro Glu Glu Pro Val Ser Gln Arg Ala Ser Arg Val Ala Glu
   65 70 75 80
   Gln Leu Phe Arg Lys Phe Leu Lys Phe Ala Glu Asn Val Gly His His
   85 90 95
   Ser Glu Lys Ala Phe Lys Lys Ala Lys Val Val Ala Glu Lys Gly Phe
   100 105 110
   Thr Ala Ala Lys Thr His Thr Val Arg Gly Phe Lys Val Ala Lys Glu
   115 120 125
   Ala Ala Gly Arg Gly Met Val Thr Val Gly Lys Lys Leu Ala Asn Val
   130 135 140
   Glu Ser Asp Arg Ser Thr Thr Thr Thr Gln Ala Pro Asp Ser Pro Asn
   145 150 155 160
   Gly Leu Ala Glu Thr Glu Val Pro Val Glu Pro Gln Gln Arg Ala Ala
   165 170 175
   His Val Pro Val Pro Asp Phe Ser Gln
   180 185

## Revendications

1. Fragment d'acide nucléique, **caractérisé en ce qu'**il comprend la séquence SEQ.ID.NO:1.

2. Fragment d'acide nucléique codant pour un polypeptide comprenant au moins un épitope de l'antigène GP28.5 de toxoplasme, **caractérisé en ce que** ledit polypeptide est constitué par les 8 à 15 acides aminés C-terminaux de la séquence SEQ.ID.NO:2.

3. Fragment peptidique constituant un épitope de l'antigène GP28.5 de toxoplasme, **caractérisé en ce qu'**il est constitué par les 8 à 15 acides aminés C-terminaux de la séquence SEQ.ID.NO:2.

4. Procédé de production d'un fragment peptidique selon la revendication 3, lequel procédé est **caractérisé en ce qu'**il comprend une étape au cours de laquelle on procède à la mise en culture d'une cellule eucaryote ou procaryote transformée par un fragment d'acide nucléique selon la revendication 2.

5. Composition antigénique **caractérisée en ce qu'**elle comprend au moins un fragment peptidique selon la revendication 3.

6. Procédé de préparation d'anticorps anti-GP28.5, **caractérisé en ce qu'**il comprend une étape au cours de laquelle on immunise un animal avec une composition antigénique selon la revendication 5.

7. Utilisation d'une composition antigénique selon la revendication 5 pour l'obtention de réactifs de diagnostic de la toxoplasmose.

8. Utilisation d'une composition antigénique selon la revendication 5 pour l'obtention de vaccins anti-toxoplasme.

## Patentansprüche

1. Nukleinsäurefragment, **dadurch gekennzeichnet, dass** es die Sequenz SEQ ID NO: 1 umfasst.

2. Nukleinsäurefragment, welches für ein Polypeptid codiert, das wenigstens ein Epitop des Toxoplasma-GP28.5-Antigens umfasst, **dadurch gekennzeichnet, dass** das Polypeptid aus den 8 bis 15 C-terminalen Aminosäuren der Sequenz SEQ ID NO: 2 besteht.

3. Peptidfragment, welches ein Epitop des Toxoplasma-GP28.5-Antigens darstellt, **dadurch gekennzeichnet, dass** es aus den 8 bis 15 C-terminalen Aminosäuren der Sequenz SEQ ID NO: 2 besteht.

4. Verfahren zur Herstellung eines Peptidfragments gemäß Anspruch 3, wobei das Verfahren **dadurch gekennzeichnet ist, dass** es einen Schritt umfasst, bei dem man eine Eukaryonten- oder Prokaryontenzelle, die mit einem Nukleinsäurefragment gemäß Anspruch 2 transformiert ist, kultiviert.

5. Antigene Zusammensetzung, **dadurch gekennzeichnet, dass** sie wenigstens ein Peptidfragment gemäß Anspruch 3 umfasst.

6. Verfahren zur Herstellung von Anti-GP28.5-Antikörpern, **dadurch gekennzeichnet, dass** es einen Schritt umfasst, bei dem man ein Tier mit einer antigenen Zusammensetzung gemäß Anspruch 5 immunisiert.

7. Verwendung einer antigenen Zusammensetzung gemäß Anspruch 5 zum Erhalt von Reagenzien zur Diagnose von Toxoplasmose.

8. Verwendung einer antigenen Zusammensetzung gemäß Anspruch 5 zum Erhalt von anti-Toxoplasma-Impfstoffen.

## Claims

1. Nucleic acid fragment, **characterised in that** it comprises the sequence SEQ. ID. NO:1.

2. Nucleic acid fragment encoding a polypeptide comprising at least one epitope of the toxoplasma GP28.5 antigen, **characterised in that** the polypeptide is constituted by the C-terminal 8 to 15 amino acids of the sequence SEQ. ID. NO:2.

3. Peptide fragment constituting an epitope of the toxoplasma GP28.5 antigen, **characterised in that** it is constituted by the C-terminal 8 to 15 amino acids of the sequence SEQ. ID. NO:2.

4. Process for the production of a peptide fragment according to claim 3, which process is **characterised in that** it comprises a step during whichan eukaryotic or prokaryotic cell transformed by a nucleic acid fragment according to claim 2 is cultured.

5. Antigen composition, **characterised in that** it comprises at least one peptide fragment according to claim 3.

6. Process for the preparation of anti-GP28.5 antibodies, **characterised in that** it comprises a step during which an animal is immunised with an antigen composition according to claim 5.

7. Use of an antigen composition according to claim 5 for the production of diagnostic reagents for toxoplasmosis.

8. Use of an antigen composition according to claim 5 for the production of anti-toxoplasma vaccines.
